## Europäisches Patentamt
## European Patent Office
## Office européen des brevets

(11) Numéro de publication: **0 069 029**
**B1**

# FASCICULE DE BREVET EUROPÉEN

(45) Date de publication du fascicule du brevet:
13.01.88

(21) Numéro de dépôt: **82420073.7**

(22) Date de dépôt: **09.06.82**

(51) Int. Cl.⁴: **A 61 M 1/16**

(54) **Rein artificiel avec circuits intégrés.**

(30) Priorité: **16.06.81 FR 8111985**

(43) Date de publication de la demande:
**05.01.83 Bulletin 83/1**

(45) Mention de la délivrance du brevet:
**13.01.88 Bulletin 88/2**

(84) Etats contractants désignés:
**BE CH DE GB IT LI NL SE**

(56) Documents cité:
**EP-A-0 005 266**
**EP-A-0 052 571**
**DE-A-2 055 281**
**US-A-3 774 762**

(73) Titulaire: **HOSPAL INDUSTRIE, 7, Avenue Lionel Terray, F-69330 Meyzieu (FR)**

(72) Inventeur: **Vantard, Georges, 8, allée d'Alexandrie, F-77420 Champs sur Marne (FR)**
Inventeur: **Clement, Gilbert, 5, rue du Château d'eau Bonville, F-28700 Auneau (FR)**
Inventeur: **Vasseur, Jean- Pierre, 19, Square Maurice Ravel, F-91160 Longjumeau (FR)**

(74) Mandataire: **Gauckler, Jacques, Service Brevets HOSPAL HOSPAL C.O.T. B.P. 21, F-69881 Meyzieu Cedex (FR)**

EP 0 069 029 B1

## Description

La présente invention concerne un rein artificiel. Elle concerne plus particulièrement un rein artificiel comportant divers organes incorporés dans un ensemble commun à usage unique.

On a déjà decrit dans le US-A-3 774 762 un rein artificiel dont la plupart des organes sont regroupés et même integrés dans un ensemble commun comportant entre des panneaux supports rigides deux larges feuilles à usage unique, conformmées selon un schéma de circulation des fluides déterminée et scellées ensemble. Cet ensemble est raccordé d'une part au patient par des conduits directs dépourvus d'accessoires et d'autre part à un pupitre où sont regroupés les organes de commande et de contrôle.

Il permet des coûts de fabrication réduits et une mise en oeuvre plus sûre et plus rapide. Cependant les différents circuits étant étalés, avec tous les réservoirs de liquide, sur une très faible épaisseur, ils occupent une surface considérable. En outre ce rein artificiel ne permet pas de réguler automatiquement la pression du liquide de dialyse dans l'hémodialyseur, ni de prélever des quantités prédéterminées d'ultrafiltrat.

Aussi est-ce un objet de la présente invention de proposer un rein artificiel qui permette, outre les opérations de dialyse classique, une meilleure maîtrise de l'ultrafiltration du sang.

Un autre objet de la présente invention est un rein artificiel de construction simplifiée, compacte, mettant en jeu moins de pièces et moins de matière, donc sensiblement plus économique.

Un autre objet de la présente invention est un rein artificiel de faible poids et de faible encombrement, facilitant ainsi son transport, son stockage et son utilisation.

Un autre objet de la présente invention est un rein artificiel qui réduise sensiblement les manipulations et les erreurs possibles, qui supprime les risques dûs à la stérilisation et dont la mise en oeuvre soit ainsi à la fois rapide, sûre, simple et économique, et particulièrement bien adaptée à la dialyse à domicile.

D'autres objets de la présente invention apparaîtront au cours de la description qui va suivre.

Il a été maintenant trouvé un rein artificiel comprenant:

- un hémodialyseur (a) divisé par une membrane permettant le traitement du sang notamment par dialyse et par ultrafiltration en deux compartimens, le premier parcouru par le sang et le second par le liquide de dialyse,
- des moyens (b) constituant le circuit extracorporel parcouru par le sang à l'extérieur dudit hémodialyseur,
- des moyens (c) pour préparer le liquide de dialyse,
- des moyens (d) pour faire circuler le liquide de dialyse,
- des moyens (f) pour stocker ledit liquide de dialyse, frais et/ou usagé,
- des organes de commande et de contrôle (h), notamment desdits moyens (b) et (d),

lesdits moyens a, b, c, d et f étant au moins partiellement intégrés dans un ensemble commun (D) constitué d'éléments obtenus par moulage ou par injection, lesdits éléments comprenant au moins deux éléments flexibles constitués chacun par un diaphragme étanche, mince de forme générale plane et comprenant des éléments multifonctionnels rigides ou semi-rigides,

caractérisé en ce qu'il comprend :
- des moyens (e) pour réguler la pression du liquide de dialyse dans ledit hémodialyseur (a),
- des moyens (g) pour prélever et mesurer des quantités de liquide égales aux quantités d'ultrafiltrat désirées, en ce que lesdits moyens (e) et (g) sont au moins partiellement intégrés dans ledit ensemble commun (D) et en ce que lesdits éléments flexibles sont munis d'ouvertures permettant aux liquids de les traverser et sont situés de part et d'autre d'au moins un desdits éléments multifonctionnels rigides ou semi-rigides et entre au moins deux autres de cesdits éléments, cesdits éléments rigides ou semi-rigides ménageant entre eux et de part et d'autre de chacun desdits éléments flexibles, les circuits de sang et de liquide de dialyse, les dits éléments flexibles assurant notamment, en coopération avec lesdits éléments rigides, des fonctions d'étanchéité.

Le présente invention n'est pas fondée sur une recherche de la meilleure façon possible d'assembler toutes les pièces nécessaires à la réalisation des fonctions souhaitées, mais au contraire sur une recherche de la réduction du nombre d'organes autonomes par incorporation dans un ensemble commun d'organes différents ayant chacun au moins une fonction particulière.

Selon l'invention on résoud ce problème par une intégration au moins partielle des moyens constituant le circuit extracorporel parcouru par le sang à l'extérieur de l'hémodialyseur, ainsi que des moyens de circulation du liquide de dialyse.

Par intégration, on entend ici l'emploi d'éléments multifonctinnels, communs à au moins deux organes différents et ayant dans chacun de ces organes une fonction particulière. Ces fonctions peuvent être soit de même nature, soit de nature différente.

Par intégration totale, on entend un ensemble regroupant divers organes constitués exclusivement d' éléments multifonctionnels.

Par intégration partielle, on entend soit un ensemble constitué d'organes dont une partie seulement a en commun un ou plusieurs éléments multifonctionnels, soit un ensemble constitué d'organes qui ne sont que partiellement constitués d' éléments multifonctionnels, soit encore un ensemble constitué par une combinaison des deux cas précédents.

Par ailleurs, comme les hémodialyseurs ont jusqu'à présent bénéficié de nombreux et importants perfectionnements justifiés par des fabrications en grande série et comme l'appareillage ayant notamment pour fonction de préparer, de stocker et de faire circuler le liquide de dialyse, ainsi que de contrôler l'ultrafiltrat n'a, par contraste, que relativement peu évolué, il en résulte un sensible déséquilibre technologique. La présente invention propose donc de transférer le plus possible d'éléments de cet appareillage vers une unité à usage unique, le plus souvent construite autour de l'hémodialyseur, pour lui faire bénéficier des technologies de grande série. L'appareillage peut être ainsi considérablement allégé et simplifié grâce à une utilisation poussée des matériaux consommables, généralement à usage unique.

Il apparaîtra clairement dans la description qui va suivre que le circuit de dialyse d'un rein artificiel étant constitué d'une pluralité d'organes assurant chacun des fonctions très particulières, l'intégration dans un ensemble commun de deux ou de plusieurs de ces organes peut se prêter à de multiples solutions, d'abord par le fait de la nature et du nombre des organes intégrés, ensuite par le choix de la solution technologique retenue pour réaliser cette intégration.

On ne décrira donc ici qu'à titre d'exemples illustratifs des modes de réalisation de la présente invention et l'on citera diverses variantes possibles, la portée de la présente invention n'étant en rien limitée ni à ces exemples, ni à ces variantes.

La compréhension de l'invention sera facilitée par les figures ci-jointes qui représentent schématiquement et sans échelle déterminée divers aspects d'exemples de réalisation. Les mêmes numéros désigneront des organes identiques ou équivalents dans les différentes figures.

La figure 1 est la vue schématique d'un rein artificiel selon l'art antérieur.

La figure 2 est la vue schématique d'un rein artificiel selon l'art antérieur, dans lequel les moyens selon a et b, qui constituent l'hémodialyseur et le circuit extracorporel de sang, et qui sont généralement à usage unique, ont été regroupés dans un ensemble commun.

La figure 3 est la vue schématique d'un rein artificiel selon la présente invention, dans lequel les moyens selon b et d qui constituent les éléments essentiels des circuits parcourus par le sang et par le liquide de dialyse, sont au moins partiellement intégrés dans un ensemble commun.

La figure 4 est la vue schématique d'un mode de réalisation particulier d'un rein artificiel selon la présente invention dans lequel les organes de commande et de contrôle selon h sont groupés dans une console réutilisable à l'intérieur de laquelle ne pénètrent plus ni le circuit sang, ni le circuit de liquide de dialyse.

La figure 5 est la vue schématique d'un mode de réalisation préférentiel d'un rein artificiel selon la présente invention, dans lequel les moyens selon a, b, c, d, e, f et g tels que définis dans la présente invention sont au moins partiellement, intégrés dans un ensemble commun D.

La figure 6 est la vue schématique d'un exemple de réalisation de l'ensemble commun D à usage unique selon la figure 5, constitué par un hémodialyseur (a) du type à membrane plane, coopérant avec deux éléments flexibles (14, 15) et quatre éléments rigides (10, 11, 12 et 13) multifonctionnels .

La figure 7 est le schéma de principe d'un exemple de circuits parcourus par le sang et par le liquide de dialyse, pouvant être au moins partiellement intégrés dans un ensemble commun D tel que représenté figures 5 ou 6.

La figure 8 est le schéma en perspective des circuits parcourus par le sang et par le liquide de dialyse représentés figure 7 et constituant effectivement l'ensemble commun D.

La figure 9 est une vue éclatée, en perspective, de l'ensemble commun D tel que représenté schématiquement figure 6 et dont les différents éléments ménagent les circuits représentés en perspective figure 8.

La figure 10 est la vue partielle, en coupe selon XX, du circuit sang délimité par les éléments 12, 13 et 15 représentés figure 9.

La figure 11 est une vue éclatée, en perspective d'un autre mode de réalisation de l'ensemble commun D comprenant notamment un hémodialyseur à fibres creuses.

La figure 12 est la vue partielle, en coupe selon XII, XII, des circuits d'entrée du sang et de sortie du liquide de dialyse à l'extrémité supérieure de l'hémodialyseur à fibres creuses représenté figure 11.

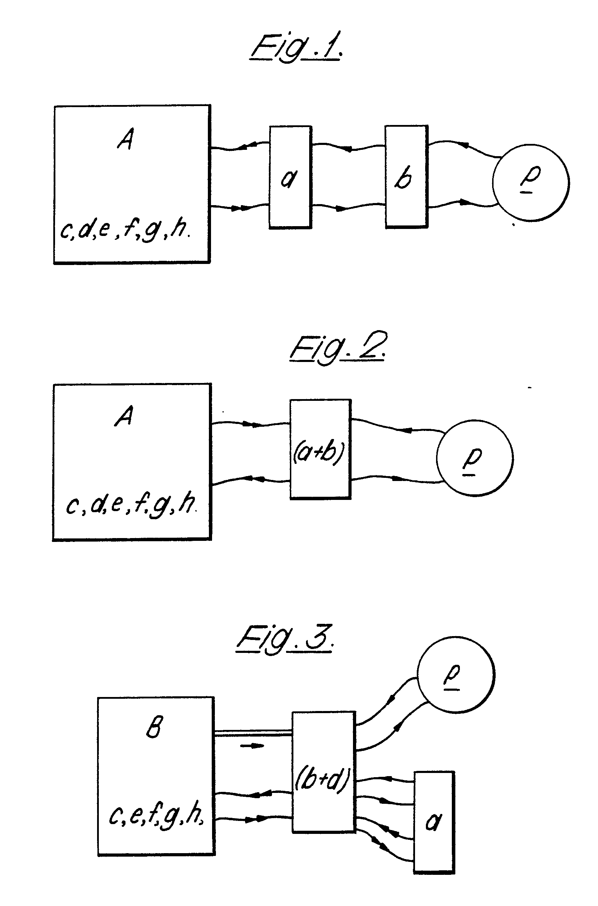

Les principaux moyens constituant un rein artificiel selon l'art antérieur sont schématisés figure 1. Le patient P est relié à un hémodialyseur (a) par des lignes parcourues par le sang et comportant différents organes tels que pompe de circulation, filtre, piège à bulles, ... figurés schématiquement par le rectangle (b). L'hémodialyseur (a) est lui-même relié à un appareil (A) comportant les moyens désignés selon la définition de la présente invention par (c), (d), (e), (f), (g) et (h).

L'appareil (A) est réutilisable après chaque traitement, moyennant une opération préalable de stérilisation. L'hémodialyseur ( a) et les lignes sang ( b) sont en général au contraire à usage unique. Aussi a-t-on déjà pensé les regrouper en les intégrant dans un ensemble commun ( a + b ) représenté figure 2.

Contrairement à l'enseignement de l'art antérieur le plus proche, on constitue selon la présente invention (cf. figure 3) un ensemble commun avec l'ensemble des circuits parcourus hors de l'hémodialyseur par le sang (b) et par le liquide de dialyse (d), ces circuits étant en outre au moins partiellement intégrés l'un à l'autre, c'est-à-dire constitués matériellement au moins en partie d'éléments communs multifonctionnels. L'ensemble commun groupant les moyens (b + d) est conçu pour un usage unique. Par ailleurs, il est relié d'une part au patient P, d'autre part à l'hémodialyseur (a), enfin à l'appareil réutilisable simplifié B qui ne comporte plus que les moyens selon c, e, f, g et h.

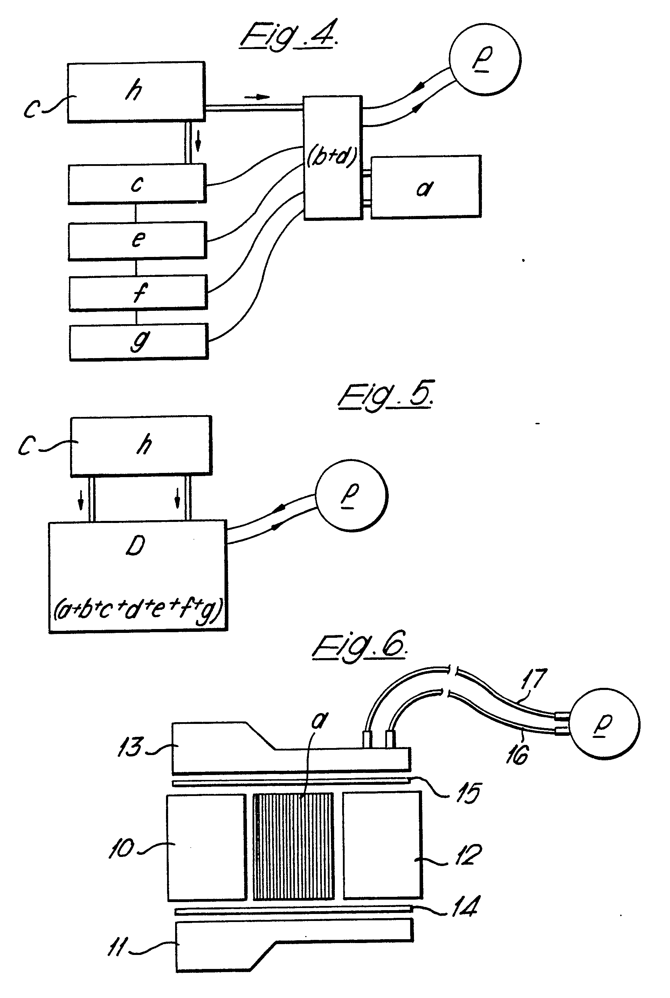

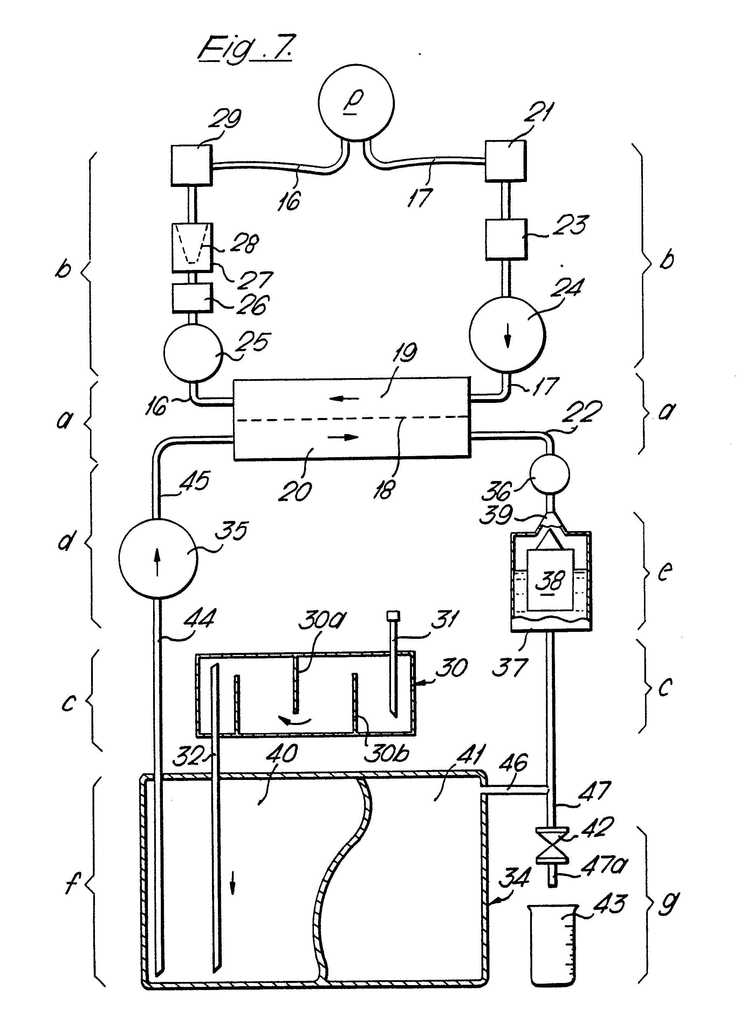

La présente invention, comme on le verra par la suite, se prête à de multiples modes de réalisation, tel celui représenté par exemple figure 4. On rearque notamment que l'hémodialyseur (a) est juxtaposé à l'ensemble comun (b + d), c' est-à-dire qu'il est directement relié à celui-ci par des connexions courtes, rigides et de préférence aisément verrouillables. On remarque en outre que les organes de commande et de contrôle (h) sont groupés dans un boîtier (C) à l'intérieur duquel ne pénètre plus ni le circuit sang, ni le circuit de liquide de dialyse. Ce boîtier ne regroupe plus que des organes électriques, mécaniques ou électroniques, et le cas échéant, des circuits hydrauliques auxiliaires, ce qui constitue une importante simplification et offre we sécurité d'emploi améliorée. Les moyens selon c, e, f et g peuvent être autonomes et sont reliés entre eux, à l'ensemble commun (b + d) et le cas échéant au boîtier de commande C.

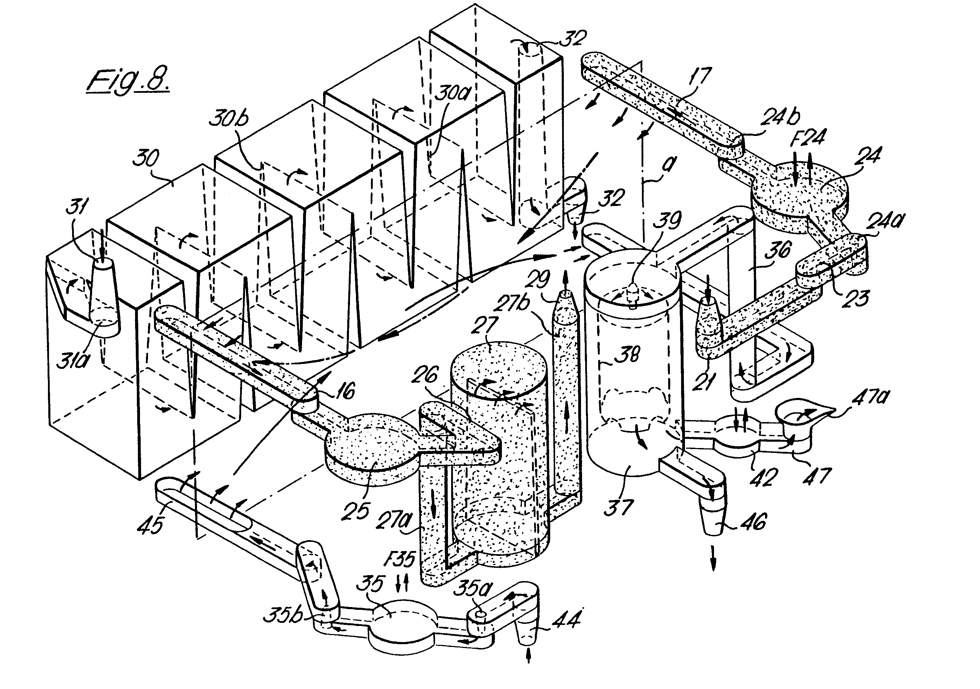

Figure 5, un ensemble commun D, à usage unique, regroupe l'ensemble des moyens désignés par a, b, c, d, e, f et g selon la présente invention. Ces moyens sont au moins partiellement intégrés les uns aux autres. Il suffit, pour constituer le rein artificiel selon l'invention de relier l'ensemble commun D au boîtier C réutilisable et réunissant les organes de commande et de contrôle, et, pour mettre en oeuvre ce rein, de relier l'ensemble commun D au patient P.

C'est ce mode de réalisation préférentiel qui sera décrit maintenant plus en détails. Ainsi la figure 6 représente schématiquement un ensemble commun D comportant les moyens a, b, c, d, e, f et g, et capable d'assumer toutes les fonctions correspondantes. Il est constitué d'un hémodialyseur (a) du type à membrane plane coopérant avec quatre éléments rigides multifonctionnels (10), (11), (12), (13) et deux éléments flexibles multifonctionnels (14) et (15) séparés et indépendants l'un de l'autre. Il peut être relié au patient P par deux simples conduits soupies (16) et (17) pour la circulation du sang, dépourvus de tout organe particulier.

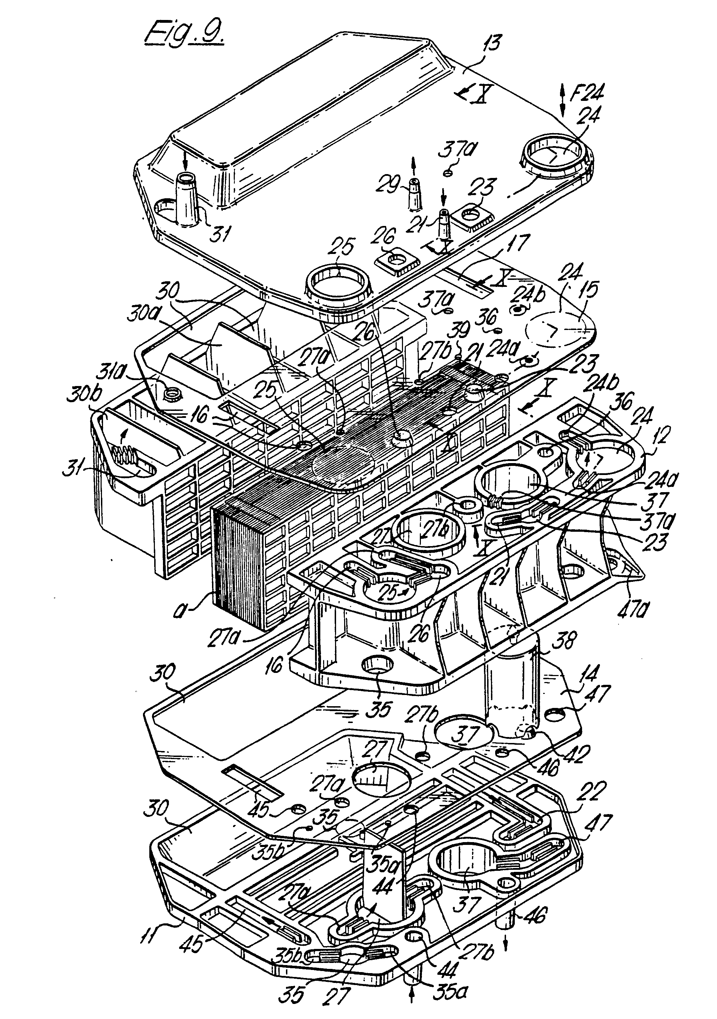

La figure 7 illustre en détails et de façon schématique sur un exemple les différents moyens a, b, c, d, e, f, et g qui sont regroupés et au moins partiellement intégrés dans un ensemble commun D selon les figures 5 et 6, ainsi que leurs liaisons.

Ainsi l'hémodialyseur (a) est divisé par une membrane (18) permettant le traitement du sang, notamment par dialyse et par ultrafiltration, en deux compartiments (19) et (20), le premier parcouru par le sang et le second par le liquide de dialyse.

Les moyens (b) constituant le circuit extracorporel parcouru par le sang à l'extérieur de l'hémodialyseur, comprennent un conduit artériel (17) et un conduit veineux (16) directement raccordés au patient et sur lesquels, à l'intérieur de l'ensemble commun D, on trouve successivement une série de dispositifs exerçant chacun une fonction spécifique, connue en soi. Ces dispositifs comprennent par exemple sur la branche artérielle (17) une tétine d'entrée sang (21), une zone de piqûre (23), permettant le cas échéant de pratiquer des injections de sérum, une pompe de circulation du sang (24) et, sur la branche veineuse (16), des moyens (25) de mesure de la pression du sang, une zone de piqûre (26) , un piège à bulles (27), un filtre à sang (28) et une tétine de sortie (29).

Les moyens (c) pour préparer le liquide de dialyse comprennent essentielleent un réservoir (30) contenant initialement une solution concentrée de liquide de dialyse dont le volume correspond exactement à la capacité en liquide de dialyse de la totalité du circuit ce qui rend superflu l'emploi d'un conductivimètre. Un conduit (31) permet l'introduction d'eau, généralement portée préalablement à une température comprise entre 37° C et 40° C et adoucie. Un tube plongeur (32) relie la partie supérieure du réservoir (30) au fond du container de stockage (34).

Des chicanes (30 a) et (30 b) peuvent favoriser le déplacement régulier du concentré par l'eau.

Les moyens (d) comprennent essentiellement une pompe de circulation (35) pour le liquide de dialyse, ainsi que les conduits de liaison tels que (44) et (45) entre d'une part le compartiment (40) de stockage de liquide de dialyse frais et l'hémodialyseur et d'autre part, tels que (22) et (46) entre l'hémodialyseur et le compartiment (41) de stockage de liquide de dialyse usagé. Ils comportent également des accessoires disposés habituellement sur ce circuit, tels par exeple sur le conduit (22) une portion transparente (36) qui coopère avec un colorimètre (non représenté) disposé sur la console (h). Naturellement ils pourraient comporter le cas échéant un conductivimètre et/ou des moyens de préchauffage du liquide de dialyse.

Les moyens (e) pour réguler la pression du liquide de dialyse dans l'hémodialyseur sont constitués dans l'exemple décrit par un bac (37) contenant un flotteur (38), le somet du flotteur coopérant avec l'orifice d'entrée (39) du bac pour réguler automatiquement, de manière connue en soi, la pression du liquide de dialyse dans l'hémodialyseur.

Les moyens (f) comprennent, à l'intérieur d'un container de stockage (34), avantageusement calorifugé, une poche plastique souple, déformable, étanche, initialement vide d'air et susceptible d'occuper un volume égal au volume interne du container. Elle est divisée en deux compartiments complémentaires (40) et (41). Le compartiment (40) est relié au réservoir de concentré (30) par le tube plongeur (32) et à la pompe (35) par un conduit (44); il contient le liquide de dialyse frais. Le compartiment (41) est relié au bac (37) par le conduit (46) d'où il reçoit le liquide de dialyse usagé.

Les moyens (g) comprennent par exemple un dispositif d'obturation (42) du conduit (47) et un récipient gradué (43) permettant de prélever et de mesurer, de manière connue en soi, des quantités de liquide, essentiellement du liquide de dialyse usagé, égales aux quantités d'ultrafiltrat désirées.

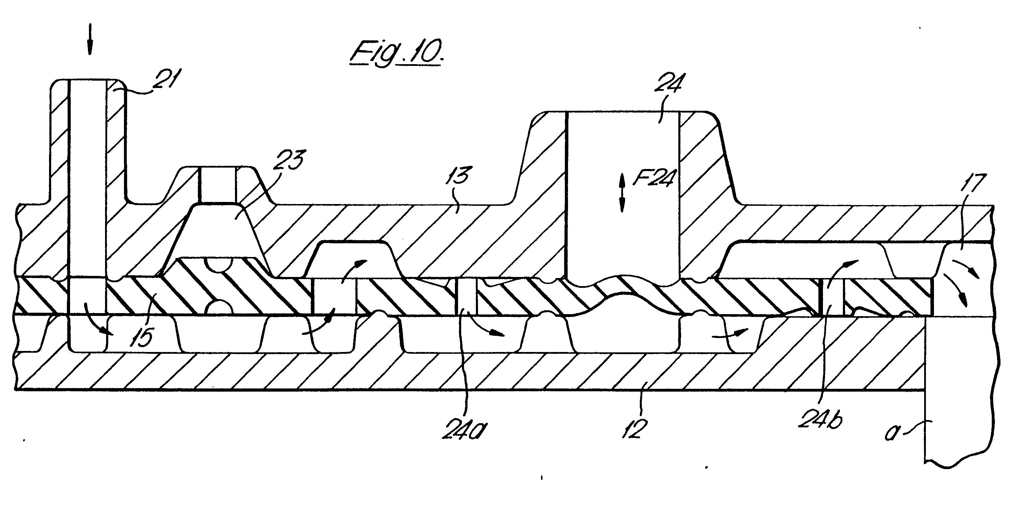

Si l'on se réfère maintenant aux figures 8 et 10, on voit que le sang artériel pénètre dans une tétine d'entrée sang (21), traverse la zone de piqûre (23) permettant des injections de sérum, puis atteint la pompe de circulation (24) par example d'un type à membrane plane connu en soi, dont le mouvement alternatif est commandé pneumatiquement ou mécaniquement selon les flèches F 24 par un moteur (non représenté) compris dans les moyens (h). La pompe (24) est disposée entre deux clapets anti-retour à membrane (24 a) et (24 b) également de types connus en soi. Le sang est ainsi pulsé dans le conduit artériel (17) vers une extrémité supérieure de l'hémodialyseur (a) représenté schématiquement figure 8 par un rectangle en perspective.

Le sang sort de l'extrémité supérieure opposée de l'hémodialyseur par le conduit veineux (16), passe sous la membrane en une zone (25) susceptible d'être reliée à un manomètre ou à un capteur de force de type connu en soi logé sur la console (h), traverse une zone de piqûre (26), puis gagne par un conduit (27 a) un piège à bulles (27) de type connu en soi comportant des moyens de mise à la pression atmosphérique et, à sa base, une filtre à sang d'un type classique (non représentés). Le sang gagne enfin par le conduit (27 b) la tétine de sortie (29) d'où il retourne au patient.

La solution concentrée de liquide de dialyse est stockée dans le réservoir (30) compartimenté par une série de chicanes verticales alternées (30 a) et (30 b). L'eau préalablement préchauffée et adoucie est introdite par le conduit (31) et traverse un clapet anti-retour (31 a) analouge au clapet (24 a) de la pompe (24). Le mélange d'eau et de solution concentrée est évacué par le tube plongeur (32) (partiellement représenté) dans le compartiment (40) du container de stockage 34 (cf. figure 7).

Le liquide de dialyse frais est repris de ce compartiment (40) via le conduit (44) par une pompe à membrane plane (35) d'un type connu en soi, analouge à la pompe (24) et dont le mouvement alternatif est commandé selon les flèches F 35 par un moteur (non représenté) compris dans les moyens h. La pompe est disposée entre deux clapets anti-retour (35 a) et (35 b), analogues aux clapets (24 a) et (24 b). Le liquide de dialyse est ainsi refoulé par le conduit (45) vers une extrémité inférieure de l'hémodialyseur (a) qu'il traverse à contre-courant du sang. Il sort à l'extrémité inférieure opposée de l'hémodialyseur pour traverser un tube transparent (36) coopérant avec un colorimètre (non représenté) logé dans la console (h) et atteindre l'orifice (39) du bac (37) contenant le flotteur (38). Le conduit (46) ramène le liquide de dialyse usagé au compartiment (41).

Le conduit (47) permet, grâce au dispositif d'obturation (42) de recueillir un volume de liquide égal au volume d'ultrafiltrat désiré qui s'écoule par le bec verseur (47 a) dans un récipient gradué (43).

L'hémodialyseur (a) représenté figure 9, est constitué de manière connue en soi par un empilement d'intercalaires et de mebranes planes pliées autour de ces intercalaires. Ceux-ci sont constitués soit de plaques pleines soit de treillis ajourés.

Les éléments flexibles (14) et (15) assurent notament les fonctions d'étanchéité, respectivement au contact du liquide de dialyse et du sang, à la fois dans l'hémodialyseur et en dehors de celui-ci.

Les éléments rigides ou semi-rigides (10), (11), (12) et (13) exercent ensemble une fonction de contention vis-à-vis de l'empilement d'intercalaires et de membranes constituant l'hémodialyseur. Ainsi on procède d'abord au serrage de l'hémodialyseur (a) entre les éléments latéraux (10) et (12), soit à cote constante, soit à pression constante, puis, ces éléments étant maintenus en place, on procède ensuite à la mise en place des éléments flexibles (14) et (15), puis des éléments (11) et (13) que l'on assemble par exemple par soudage aux ultra-sons sur les éléments (10) et (12).

On se rend compte ainsi que le montage de l'ensemble commun D selon l'invention se ramène au simple montage des plaques d'extrémités et des plaques latérales d'un hémodialyseur. Ceci est un avantage remarquable compte tenu des très nombreuses fonctions supplémentaires exercées par l'ensemble commun D par rapport à un hémodialyseur classique.

L'élément inférieur multifonctionnel (11) comprend essentiellement une cuvette constituant le fond du bac (30) de solution concentrée et un orifice (non représenté) permettant le passage du tube plongeur (32). Il comprend en outre des canaux (44), (45) pour la circulation du liquide de dialyse frais, le logement de la pompe à membrane (35) pour la circulation du liquide de dialyse et de ses clapets (35 a) et (35 b), les canaux d'évacuation (22) du liquide de dialyse usagé et le fond du bac (37). Il comprend enfin le fond du piège à bulles (27) pour le sang.

L'élément flexible inférieur (14) assure notament une fonction d'étanchéité en particulier sur le circuit du liquide de dialyse, étant serré par l'élément inférieur (11) contre les éléments (10), (12), et (a). Il est en outre percé des orifices suivants:

- découpe autour du bac (30) de solution concentrée de dialyse,
- découpes (44) et (45) permettant au liquide de dialyse frais de pénétrer dans l'hémodialyseur après traversée de l'élément inférieur (11).

- découpes autour du piège à bulles (27) et conduits sang amont et aval (27 a) et (27 b).
- découpes autour du bac (37) recevant le liquide de dialyse usagé.
- découpes des orifices (35 a) et (35 b) permettant à cet élément flexible de constituer la partie active des clapets correspondants de la pompe (35).

Cet élément flexible constitue en outre la partie active de la pompe à membrane (35) pour la circulation du liquide de dialyse.

Les moyens de stockage (f) du liquide de dialyse sont constitués à l'intérieur du container (34) par les deux compartiments (40) et (41) d'une poche souple. Ces compartiments peuvent être formés par exemple à partir d'une gaine tubulaire extrudée en polyéthylène, thermosoudée aux deux extrémités et raccordée aux orifices et tubulures correspondants (32, 44 et 46).

Ces moyens de stockage sont intégrés au moins partiellement dans ledit ensemble commun D par le fait que l'élément inférieur multifonctionnel (11) peut constituer le couvercle du container (34). La gaine tubulaire aplatie couvre alors la face inférieure de l'élément (11). Cette intégration peut être encore accrue par le fait que l'on peut avantageusement introduire la gaine tubulaire aplatie, avec l'élément flexible (14), entre les éléments rigides ou semi-rigides (11) d'une part, (10, 12 et a) d'autre part, les orifices appropriés correspondants pouvant être exécutés à la fois dans l'élément flexible (11) et dans l'une au moins des parois de la gaine tubulaire aplatie.

L'élément latéral multifonctionnel (10) comprend essentiellement une cavité allongée (30), ouverte vers le haut et vers le bas, nervurée intérieurement par des éléments (30 a) et (30 b) jouant à la fois le rôle de chicanes et d'éléments raidisseurs. Il prend appui directement sur l'empilement de mebranes et d'intercalaires constituant l'hémodialyseur (a) par une face convenablement nervurée. Il comprend en outre un élément du tube plongeur (32) et un logement pour le clapet anti-retour (31 a).

L'élément latéral multifonctionnel (12) occupe une position sensiblement symétrique de l'élément (10) précédent par rapport à l'hémodialyseur (a). Toutefois il réunit la majorité des organes disposés sur le circuit sang et sur le circuit de dialyse usagé. C'est ainsi qu'il comprend (voir aussi fig. 10) le conduit artériel amenant le sang de la tétine d'entrée sang (21) à la zone de piqûre (23) et de là à la pompe à membrane (24). Il comprend le corps de la pompe (24) et les logements correspondants aux clapets amont et aval, respectivement (24 a) et (24 b).

Sur le circuit de liquide de dialyse usagé, il comprend le conduit (36) alimentant le bac (37) depuis l'orifice supérieur (39), le bac (37) lui-même, à l'intérieur duquel vient se loger le flotteur (38). Le canal (37 a) met la partie supérieure du bac (37) en communication avec l'atmosphère. La face inférieure de l'élément (12) est rainurée de façon à offrir en coopération avec les éléments inférieurs (14) et (11) les circuits déjà décrits. L'orifice (35) permet une liaison mécanique entre la console (h) et la membrane de la pompe (35) de circulation du liquide de dialyse.

Les moyens (g) pour prélever et mesurer des quantités (en volume, en poids et/ou en débit) de liquide égales aux quantités d'ultrafiltrat désirées sont constitués par un conduit (47) muni d'une section souple (42) susceptible d'être fermée par un dispositif extérieur de tout type connu tel qu'une vis réglable qui agit selon la flèche (F 42) pour obturer plus ou moins la section du conduit (47). Ce conduit (47) s'ouvre par un bec verseur (47 a) dans un récipient gradué (43). Le volume de liquide soutiré est aussitôt remplacé par un égal volume d'ultrafiltrat traversant la membrane jusqu'à ce que la pression du liquide de dialyse dans l'hémodialyseur soit rétablie à sa valeur initiale. Le rein artificiel selon l'exemple décrit contrôle donc l'ultrafiltrat par une méthode volumétrique.

L'élément fiexible supérieur (15) assure notamment une fonction d' étanchéité, en particulier sur le circuit sang, étant serré par l'élément supérieur (13) contre les éléments (10), (12), et (a).

Il est en outre muni notamment des orifices suivants:
- découpe autour du bac (30) de solution concentrée ce dialyse,
- clapet (31 a) pour l'introduction d'eau dans le bac (30),
- découpes (16) et (17) pour l'introduction et l'évacuation du sang dans l'hémodialyseur (a),
- orifices (24 a) et (24 b) permettant à cet élément flexible de constituer la partie active des clapets correspondants de la pompe (24),
- les orifices (36) et (39) permettant au liquide de dialyse usagé de pénétrer à la partie supérieur du bac (37) contenant le flotteur (38),
- les orifices (27 a) et (27 b) correspondant aux conduits sang respectivement à l'amont et à l'aval du piège à bulles (27).
- l'orifice (37 a) de mise à l'atmosphère du bac (37).

Cet élément flexible comporte en outre des sections renforcées au droit des zones de piqûres (23) et (26), permettant le cas échéant de pratiquer des injections de sérum. Cet élément flexible (15) constitue également la partie active de la pompe à membrane (24) pour la circulation du sang, ainsi que la partie active du capteur de pression (25).

L'élément supérieur multifonctionnel (13) comprend essentiellement un bossage recouvrant le bac (30) de solution concentrée, un conduit (31) pour l'introduction d'eau, les deux tétines sang (21) et (29), deux orifices (23) et (26) correspondant aux zones de piqûres, respectivement à l'amont et à l'aval de l'hémodialyseur (a), 2 orifices (24) et (25) correspondant au dégagement nécessaire de la membrane au droit de la pompe à sang et de la prise de pression veineuse et l'orifice (37 a) de mise à l'atmosphère du bac (37).

La face inférieure de l'élément (13) est rainurée de façon à offrir en coopération avec les éléments inférieurs (12) et (15) les circuits déjà décrits et illustrés notamment figure 10.

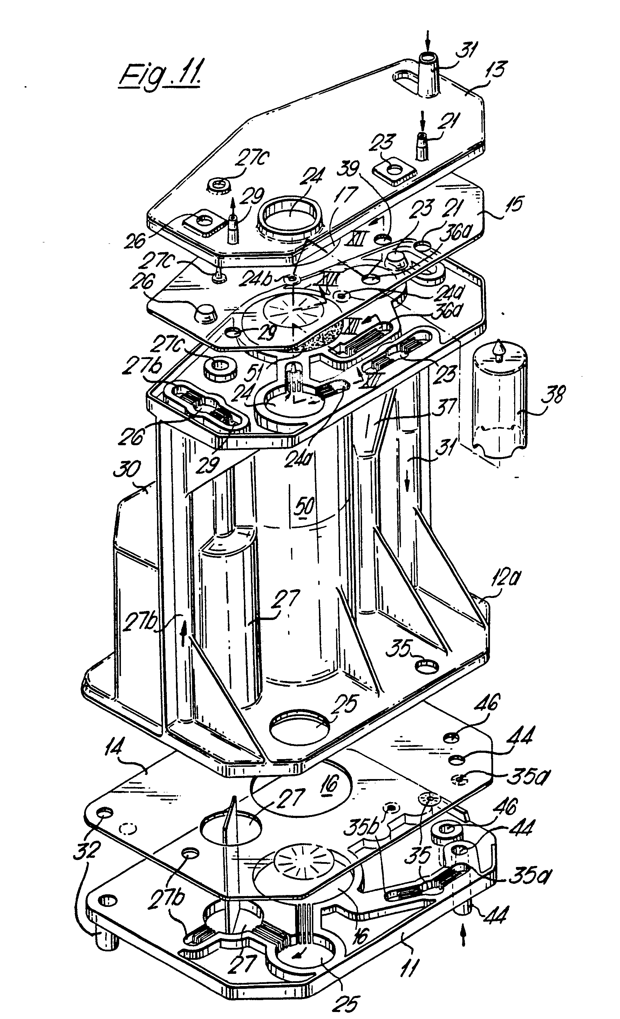

La figure 11 représente un autre mode de réalisation de l'ensemble commun intégré selon l'invention. Cet ensemble se distingue de celui représenté figure 9 essentiellement par le fait qu'il comprend un hémodialyseur du type à fibres creuses; en outre le faisceau de fibres creuses est disposé verticalement à l'intérieur d'un élément unique (12 a) qui se substitue aux éléments (10) et (12). Toutefois les autres éléments, flexibles, tels que (14) et (15), ou rigides, tels que (11) et (13), sont analogues à ceux représentés figure 9. De même, le schéma de circulation du sang et du liquide de dialyse. est d'un type comparable à celui représenté figure 8, les mêmes numéros désignant les mêmes organes ou des organes équivalents assurant les mêmes fonctions.

L'élément (12 a), moulé d'une seule pièce, comporte dans sa partie médiane une chambre cylindrique (50) ouverte à ses extrémités inférieure et supérieure. Un faisceau de fibres creuses (51) est introduit à l'intérieur de la chambre (50) et dépasse des deux côtés. Les extrémités des fibres creuses sont scellées entre elles et aux parois intérieures de la chambre (50) de manière connue en soi par introduction d'une résine solidifiable (52) par exemple à base de composés polyuréthanes, et centrifugation. Les deux extrémités du faisceau sont alors arasées dans les plans parallèles des faces délimitant l'élément (12 a), les fibres creuses étant ainsi ouvertes à leurs extrémités.

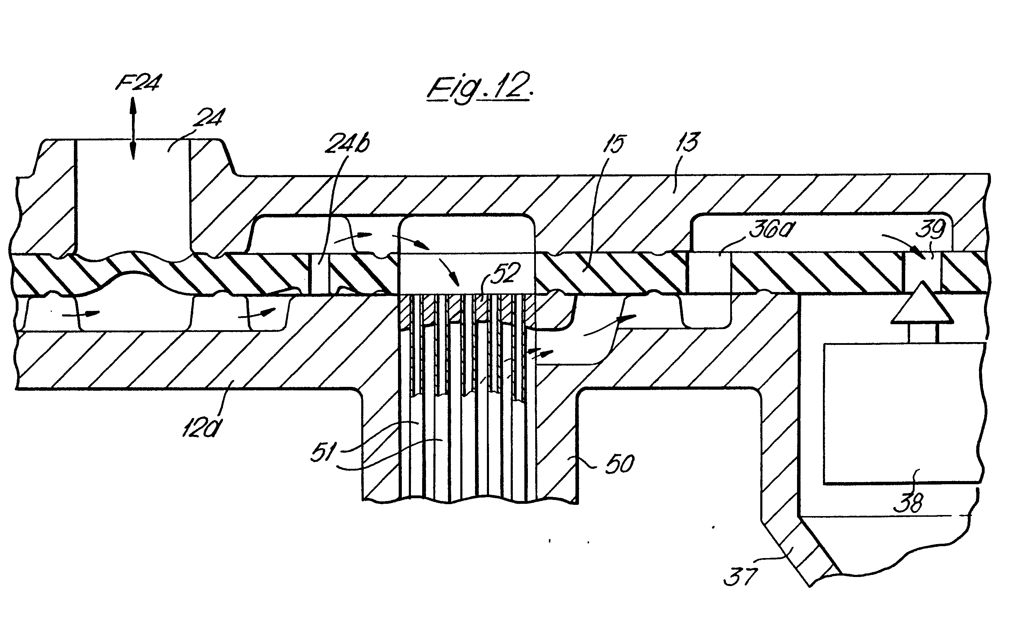

La figure 12 illustre en détails les portions de circuits parcourus par le sang et par le liquide de dialyse situés au voisinage de l'extrémité supérieure de l'hémodialyseur à fibres creuses. Des dispositions analogues existent à l'extrémité inférieure. Le sang est pulsé par la pompe à membrane (24) animée par les forces F 24 commandées par un moteur (non représenté) disposé dans la console (h) à travers un clapet anti-retour (24 b). De là il pénètre à l'intérieur des fibres creuses constituant le faisceau (51).

Le liquide de dialyse circule dans la chambre (50) à l'extérieur des fibres creuses et à contre-courant du sang, il sort latéralement sous la zone d'empotage (52) par un canal qui le conduit à l'orifice (36 a) qu'il traverse et d'où il gagne par l'orifice (39) le bac (37) contenant le flotteur (38). Avantageusement une partie au moins de la paroi de l'élément (13) délimitant le conduit entre les orifices (36 a) et (39) est en matériau transparent, coopérant avec un colorimètre (non représenté) logé dans la console (h).

Quelques variantes de réalisation sont représentées figure 11. Ainsi le bac de concentré (30) ne comporte pas de chicanes internes (30 a) ou (30 b).

De même les moyens (g) de prélèvement de quantités de liquide de dialyse égales aux quantités d'ultrafiltrat désirées ne sont pas intégrés dans l'ensemble commun et ne sont donc pas représentés. Le clapet (27 c), susceptible d'être commandé de l'extérieur (console h), permet de mettre le piège à bulles (27) à la pression atmosphérique.

On observe que l'ensemble commun intégré représenté figure 11 ne comporte pas plus de pièces qu'un simple hémodialyseur à fibres creuses classique. En effet, de part et d'autre du boîtier (12 a) contenant le faisceau de fibres creuses (51) sont rapportés deux couvercles (éléments 11 et 13) enserrant deux joints (éléments 14 et 15). L' assemblage de ces éléments peut se faire simplement par soudage aux ultra-sons après positionnement préalable.

En se référant par exemple aux figures 10 et 12, on voit un des deux éléments flexibles de l'ensemble D selon l'invention tel que (15) de forme générale plane et relativement mince, disposé entre les faces en regard de deux éléments rigides ou semi-rigides tels que 12, 12 a et 13. Tous ces éléments coopèrent pour former ainsi au moins une partie des circuits de sang et de liquide de dialyse, frais ou usagé.

Chaque circuit comprend des canaux ménagés dans chacune des faces en regard et délimités de manière étanche par l'élément flexible. Celui-ci est muni des ouvertures nécessaires pour permettre le passage du liquide d'une face sur l'autre de l'élément flexible. Avantageusement les canaux sont nervurés, des nervures parallèles allongées soutenant régulièrement l'élément flexible, assurant ainsi à la fois un passage suffisant au liquide et une étanchéité continue. Localement l'élément flexible peut être aminci pour accroître sa flexibilité lorsqu'il joue le rôle d'une pompe à membrane, ou au contraire renforcé lorsqu'il constitue une zone de piqûre, ou préformé lorsqu'il fonctionne en clapet anti-retour.

Au moins deux éléments flexibles indépendants tels que 14 et 15, de forme générale plane et mince sont disposés dans des plans différents, de préférence parallèles, de part et d'autre d'éléments rigides ou semi-rigides, tels que 10, 12 ou 12 a. Tous ces éléments coopèrent pour former des canaux longitudinaux. Avantageusement ces canaux peuvent être reliés entre eux par un ou plusieurs canaux transversaux tel que le canal (27 b) ménagé à l'intérieur de l'élément 12 a, figure 11.

On peut ainsi, selon l'invention, réaliser une grande diversité de circuits et les disposer de manière telle qu'ils ne se croisent pas. Avantageusement, pour éviter tout risque de fuite d'un liquide depuis l'un de ces circuits dans l'autre, par exemple de sang dans le liquide de dialyse, on peut aisément les séparer par des zones qui sont reliées, directement ou non, à l'atmosphère, (cf. par exemple les orifices (37 a) dans les éléments 13 et 15, figure 9).

Le rein artificiel selon la présente invention peut faire l'objet de très nombreuses variantes de réalisation à la portée du technicien. C'est ainsi que, ayant intégré dans un même ensemble commun les moyens selon a, b, c, d, e, f et g , il est toujours possible de n'intégrer qu'une partie de ces moyens autour des moyens b et d.

Par exemple il est possible de ne pas intégrer la cuve (30) de solution concentrée à l'ensemble commun D, d'où une simplification, notamment des éléments 10, 11 et 13. Mais on peut alors intégrer la cuve (30) au container (34) pour constituer un ensemble intégré secondaire que l'on raccorde à l'ensemble commun D principal. Une telle disposition peut offrir une souplesse d'emploi avantageuse. En outre, le volume de la cuve (30) peut, si désiré, être supérieur ou inférieur à la capacité en liquide de dialyse de la totalité du circuit.

On peut également retrancher, modifier ou ajouter des dispositifs accessoires. Ainsi on peut par exemple ajouter au circuit sang un piège à bulles à l'amont de l'hémodialyseur, ou embrocher une bouteille de sérum directement sur une zone de piqûre, ou disposer une deuxième pompe à sang à l'aval de l'hémodialyseur, permettant ainsi de régler la pression sang dans l'hémodialyseur, donc le débit d'ultrafiltrat, ou encore disposer un dispositif de fermeture alternatif des conduits sang pour emploi avec une aiguille unique.

Le dégazage du piège à bulles peut être automatique ou commandé de l'extérieur et par exemple asservi au maintien d'un niveau de sang sensiblement constant dans ce piège à bulles, etc...

On peut aussi, sur le circuit de liquide de dialyse, remplacer le dispositif de régulation à flotteur par un dispositif de régulation classique comportant une vanne reliée à un indicateur de la pression du liquide de dialyse qui maintient la valeur de cette pression comprise entre des limites prédéterminées. L'ensemble de ces moyens peut alors être intégré ou non à l'ensemble commun D. On peut alors disposer la pompe (35) de circulation du liquide de dialyse en aval de l'hémodialyseur (a).

On peut aussi remplacer le dispositif d'obturation (42) pour soutirer des quantités de liquide égales aux quantités d'ultrafiltrat désirées par une pompe à membrane encadrée de clapets anti-retour du même type que celles déjà utilisées pour déplacer le sang ou le liquide de dialyse et qui mette à profit les caractéristiques de l'élément flexible (14). On peut aussi substituer une poche souple au récipient gradué (43).

On peut également ajouter, par exemple dans la cuve (30) qui ne jouerait plus le rôle de réservoir de concentré, des cartouches de charbon actif pour épurer au moins partiellement le liquide de dialyse.

Dans ce cas la poche plastique souple à l'intérieur du container (34) peut ne plus comporter qu'un compartiment unique.

On peut encore ajouter au circuit du liquide de dialyse par exemple un conductivimètre, un dispositif de chauffage et de régulation de température du liquide de dialyse, un adoucisseur d'eau, une deuxième pompe de circulation du liquide de dialyse pour la recirculation dans l'hémodialyseur d'un mélange convenable de liquide de dialyse frais et de liquide usagé, recyclé.

L'hémodialyseur peut être de tout type connu. Par exemple le faisceau de fibres creuses peut avantageusement être divisé et réparti en faisceaux élémentaires dans des compartiments parcourus en série par le sang et/ou par le liquide de dialyse.

Les orifices d'entrée et de sortie du sang et du liquide de dialyse peuvent avoir, pour un hémodialyseur à membrane plane, des positions différentes de celles représentées figure 9.

Les éléments flexibles indépendants tels que 14 et 15 peuvent n'occuper qu'une fraction de la section de l'ensemble commun D; ils peuvent être décalés ou situés dans des plans non parallèles, par exemple perpendiculaires. Ils sont constitués par des diaphragmes souples et étanches, par exemple par des feuilles de polyuréthane, d'élastomère silicone, de chlorure de polyvinyle plastifié et/ou de caoutchouc synthétique d'une épaisseur de l'ordre de 0,3 à 3 mm, par exemple de 1 millimètre. Ils peuvent être obtenus par moulage.

Les accès de l'ensemble commun D à la console (h) de contrôle et de commande peuvent être prévus à volonté, par dessus, par dessous et/ou latéralement.

Les liaisons et/ou les connexions entre les éléments flexibles de l'ensemble commun D et les organes de commande et de contrôle groupés dans la console (h) peuvent être de tous types connus, par exemple pneumatiques ou mécaniques. La commande du déplacement de l'élément flexible peut être à simple ou à double effet. Par exemple on peut loger dans les éléments flexibles des inserts métalliques qui peuvent s'encliqueter (double effet) sur des tiges de commande entraînées par des dispositifs moteurs montés sur la console (h). On peut aussi appliquer sur les éléments flexibles des ventouses dont on commande le déplacement alternatif depuis la console (h).

Les éléments semi-rigides, de préférence rigides, peuvent être obtenus par des techniques se prêtant à une fabrication économique en grande série, par exemple par moulage ou par injection. Ils peuvent être exécutés avec deux, trois ou quatre directions de moulage.

On peut utiliser notamment toutes matières plastiques transparentes ou non, susceptibles d'être mises en oeuvre par ces techniques. On peut placer, le cas échéant, des inserts métalliques.

L'ensemble commun D est à usage unique. Mais il pourrait être éventuellement réutilisé au moins partiellement plusieurs fois après stérilisation. L'hémodialyseur pourrait alors se présenter sous forme de cartouche interchangeable, aisément connectable et déconnectable au reste de l'ensemble commun.

Un ensemble commun D tel que représenté figure 9, comportant un hémodialyseur de surface membranaire égale à 0,6 m² ne pèse ainsi que 1 kg environ, soit le même poids qu' un hémodialyseur seul, de même type, de surface membranaire égale à 1 m². Les volumes, donc les encombrements sont sensiblement dans les mêmes rapports. La présente invention permet en effet de réduire à chaque traitement le poids de matière plastique mis en jeu d'au moins 30 %, lorsqu'on comprend tous les accessoires nécessaires.

On constate ainsi la simplification considérable apportée par la présente invention, tant au niveau de la fabrication, que du transport, du stockage ou de l'utilisation.

En effet, le fabriquant ne met en oeuvre que très peu de matière, celle-ci étant utilisée à de multiples fins. Le nombre de pièces se réduit pratiquement à celui nécessaire à l'hémodialyseur seul. D'où un montage simple et facilement automatisable. En outre, les fonctions multiples des éléments rigides conduisant à des formes largement nervurées, ils ont la rigidité nécessaire pour assurer une excellente contention de l'hémodialyseur et une très bonne étanchéité des circuits sang et liquide de dialyse.

Par ailleurs, il suffit pour l'utilisateur de posséder une consoie (h) très réduite, car dépourvue notamment de tout organe correspondant au circuit de liquide de dialyse, des moyens lui permettant d'obtenir de l'eau adoucie préchauffée et un container pour recevoir le liquide de dialyse. A chaque séance, il utilise un enseble commun D qui comprend tout l'équipement complémentaire nécessaire et qu'il jette généralement après usage, afin de bénéficier des meilleures conditions de stérilité.

Ainsi, outre son économie, l'allègement considérable du matériel nécessaire, sa comodité et sa simplicité d'emploi ainsi que sa fiabilité et sa sécurité sensiblement améliorées, font du rein artificiel selon l'invention un rein particulièrement bien adapté aux exigences du traitement à domicile.

**Revendications**

1. Rein artificiel comprenant :
- un hémodialyseur (a) divisé par une membrane (18) permettant le traitement du sang notamment par dialyse et par ultrafiltration en deux compartiments (19 et 20), le premier parcouru par le sang et le second par le liquide de dialyse,
- des moyens (b) constituant le circuit extracorporel parcouru par le sang à l'extérieur dudit hémodialyseur,
- des moyens (c) pour préparer le liquide de dialyse,
- des moyens (d) pour faire circuler le liquide de dialyse,
- des moyens (f) pour stocker ledit liquide de dialyse, frais et/ou usagé,
- des organes de commande et de contrôle (h), notamment desdits moyens (b) et (d), lesdits moyens a, b, c, d et f étant au moins partiellement intégrés dans un ensemble commun (D) constitué d'éléments obtenus par moulage ou par injection, lesdits éléments comprenant au moins deux éléments flexibles (14,15) constitués chacun par un diaphragme étanche, mince de forme générale plane et comprenant des éléments multifonctionnels rigides ou semi-rigides (10, 11, 12, 13),
caractérisé en ce qu'il comprend:
- des moyens (e) pour réguler la pression du liquide de dialyse dans ledit hémodialyseur (a),
- des moyens (g) pour prélever et mesurer des quantités de liquide égales aux quantités d'ultrafiltrat désirées, en ce que lesdits moyens (e) et (g) sont au moins partiellement intégrés dans ledit ensemble commun (D) et en ce que lesdits éléments flexibles (14,15) sont munis d'ouvertures permettant aux liquids de les traverser et sont situés de part et d'autre d'au moins un desdits éléments multifonctionnels rigides ou semi-rigides (10,12) et entre au moins deux autres de cesdits éléments (11,13), cesdits éléments rigides ou semi-rigides ménageant entre eux et de part et d'autre de chacun desdits éléments flexibles, les circuits de sang et de liquide de dialyse, lesdits éléments flexibles assurant notamment, en coopération avec lesdits éléments rigides, des fonctions d'étanchéité.

2. Rein artificiel selon la revendication 1, caractérisé en ce que ledit hémodialyseur (a) est intégré audit ensemble commun (D) qui comprend deux éléments flexibles (14, 15), disposés dans des plans parallèles, situés de part et d'autre dudit hémodialyseur, lesdits éléments rigides (10, 11, 12, 13) constituant les éléments de contention des membranes dudit hémodialyseur (a).

3. Rein artificiel selon l'une des revendications 1 ou 2 , caractérisé en ce que lesdits éléments flexibles (14, 15) délimitent des portions longitudinales des circuits de sang et de liquide de dialyse et sont traversés respectivement et essentiellement, l'un par le liquide de dialyse et l'autre par le sang.

4. Rein artificiel selon la revendication 3, caractérisé en ce que lesdites portions longitudinales desdits circuits de sang et de liquide de dialyse sont reliées entre elles par au moins un conduit transversal ménagé à l'intérieur d'au moins un desdits éléments rigides ou semi-rigides (10, 12).

5. Rein artificiel selon l'une quelconque des revendications précédentes, caractérisé en ce que lesdits circuits de liquides, parcourus l'un par le sang et l'autre par le liquide de dialyse, sont séparés l'un de l'autre par une zone reliée à l'atmosphère (37 a).

6. Rein artificiel selon l'une quelconque des revendications 2 à 5 , caractérisé en ce qu'il comprend un hémodialyseur (a), de type à membrane plane, disposé entre deux éléments (14, 15) flexibles, parallèles, serrés par deux éléments rigides (11, 13) formant couvercle, sur deux autres éléments rigides (10, 12) maintenant en contention l'émpilement de membranes et d'intercalaires dudit hémodialyseur, le premier (10) de cesdits éléments de contention constituant notamment un réservoir pour le concentré de dialyse et le second (12) de cesdits éléments de contention regroupant les organes essentiels desdits circuits de sang et de liquide de dialyse.

7. Rein artificiel selon l'une quelconque des revendications 2 à 5, caractérisé en ce qu'il comprend un hémodialyseur (a) du type à faisceau de fibres creuses logé à l'intérieur d'un élément de contention rigide (12 a), dont les faces opposées parallèles sont reliées chacune à l'un desdit éléments rigides (11, 13) par l'intermédiaire de l'un desdits éléments flexibles (14, 15).

8. Rein artificiel selon l'une quelconque des revendications précédentes, caractérisé en ce que l'un quelconque desdits éléments flexibles (14, 15) constitue l'élément mobile et actif à la fois du corps de pompe (24) et des clapets (24 a, 24 b) et d'une pompe de type a diaphragme, en cooperation avec lesdits éléments rigides adjacents (10, 11, 12, 13) et avec un moteur extérieur.

9. Rein artificiel selon la revendication 8, caractérisé en ce que ledit élément flexible (14, 15) est muni de trous (24 a, 24 b) au niveau des clapets, afin d'être traversé par le liquide déplacé.


**Patentansprüche**

1. Künstliche Niere, enthaltend:
- einen durch eine Membran (18) unterteilten Hämodialysator (a), der die Behanldung des Bluts, insbesondere durch Dialyse und durch Ultrafiltration in zwei Abteilen (19 und 20) gestattet, wobei das erste vom Blut und das zweite von der Dialyseflüssigkeit durchströmt werden,
- Mittel (b), die den außerhalb des Körpers gelegenen Kreislauf bilden, der vom Blut außerhalb des Hämodialysators durchströmt wird,
- Mittel (c) zum Bereiten der Dialyseflüssigkeit,
- Mittel (d) zum Zirkulieren der Dialyseflüssigkeit,
- Mittel (f) zum Speichern der frischen und/oder verbrauchten Dialyseflüssigkeit,
- Steuer- und Kontrollorgane (h), insbesondere für die Mittel (b) und (d), wobei die Mittel (a,b,c,d und f) wenigstens teilweise in eine gemeinsame Anordnung (D) integriert sind, die aus durch Gießen oder Spritzen hergestellten Elementen bestehen, wobei diese Elemente wenigstens zwei biegsame Elemente (14,15) aufweisen, von denen jedes aus einer dünnen, dichten, insgesamt ebenen Membran besteht, die starre oder halbstarre Mehrfachfunktionselemente (10,11,12,13) enthält,
dadurch gekennzeichnet,
daß sie folgendes enthält:
- Mittel (e) zum Regeln des Drucks der Dialyseflüssigkeit im Hämodialysator (a),
- Mittel (g) zum Entnehmen und Messen von Flüssigkeitsmengen, die gleich den gewünschten Ultrafiltratmengen sind, daß die Mittel (e) und (g) wenigstens teilweise in der gemeinsamen Anordnung (D) integriert sind, und daß die biegsamen Elemente (14,15) mit Öffnungen für den Durchtritt der Flüssigkeiten versehen sind und sich beiderseits von wenigstens einem der starren oder halbstarren Mehrfachfunktionselemente (10,12) und zwischen wenigstens zwei weiteren dieser Elemente (11,13) befinden, wobei die starren oder halbstarren Elemente zwischen sich und beiderseits jedes dieser biegsamen Elemente Blut- und Dialyseflüssigkeitskreise bilden, wobei die biegsamen Elemente insbesondere in Zusammenarbeit mit den starren Elementen die Abdichtfunktionen übernehmen.

2. Künstliche Niere nach Anspruch 1, dadurch gekennzeichnet,
daß der Hämodialysator (a) in die gemeinsame Anordnung (D) integriert ist, die zwei biegsame Elemente (14,15) enthält, die in parallelen Ebenen beiderseits des Hämodialysators angeordnet sind, wobei die starren Elemente (10,11,12,13) die Umschließungselemente für die Membranen des Hämodialysators (a) bilden.

3. Künstliche Niere nach einem der Ansprüche 1 oder 2, dadurch gekennzeichnet,
daß die biegsamen Elemente (14,15) die Längsteile der Blut- und Dialyseflüssigkeitskreise begrenzen und im wesentlichen jeweils von der Dialyseflüssigkeit bzw. vom Blut durchsetzt werden.

4. Künstliche Niere nach Anspruch 3, dadurch gekennzeichnet,
daß die Längsteile der Blut- und Dialyseflüssigkeitskreise durch wenigstens eine Querleitung miteinander verbunden sind, die innerhalb wenigstens eines der starren oder halbstarren Elemente (10,12) gebildet sind.

5. Künstliche Niere nach einem der vorhergehenden Ansprüche, dadurch gekennzeichnet,
daß die Flüssigkeitskreise, die jeweils vom Blut bzw. von der Dialyseflüssigkeit durchströmt werden, durch eine mit der Atmosphäre verbundene Zone (37a) voneinander getrennt sind.

6. Künstliche Niere nach einem der Ansprüche 2 bis 5, dadurch gekennzeichnet,
daß sie einen Hämodialysator (a) von der Bauart mit ebener Membran enthält, der zwischen zwei parallelen biegsamen Elementen (14,15) angeordnet ist, die durch zwei einen Deckel bildende starre Elemente (11,13) auf zwei weitere starre Elemente (10,12) geklemmt ist, die den Stapel aus Membranen und Zwischenlagen des Hämodialysators umschlossen halten, wobei das erste (10) dieser Umschließungselemente insbesondere eimen Vorratsbehälter für das Dialysekonzentrat und das zweite (12) dieser Umschließungselemente die wesentlichen Organe der Blut- und Dialyseflüssigkeitskreise bilden.

7. Künstliche Niere nach einem der vorhergehenden Ansprüche, _dadurch gekennzeichnet,_

daß sie einen Hämodialysator (a) der Bauart mit Faserbündeln aufweist, der sich innerhalb eines starren Umschliessungselementes (12a) befindet, dessen parallele gegenüberliegenden Seitenflächen über eines der biegsamen Elemente (14,15) jeweils mit einem der starren Elemente (11,13) verbunden sind.

8. Künstliche Niere nach einem der vorhergehenden Ansprüche, _dadurch gekennzeichnet,_ daß irgendeines der biegsamen Elemente (14,15) das bewegliche und aktive Element gleichzeitig des Pumpenkörpers (24), der Ventile (24a, 24b) und einer Pumpe der Membranbauart bildet in Zusammenarbeit mit den angrenzenden starren Elementen (10,11,12,13) und mit einem außen gelegenen Motor.

9. Künstliche Niere nach Anspruch 8, _dadurch gekennzeichnet,_

daß das biegsame Element (14,15) in Höhe der Ventile mit Löchern (24a, 24b) versehen ist, um von der geförderten Flüssigkeit durchquert zu werden.

## Claims

1. Artificial kidney comprising:
- a haemodialyzer (a) divided by a membrane (18), enabling the blood to be treated, in particular, by dialysis and by ultrafiltration, into two compartments (19 and 20), the blood passing through the first compartment and the dialysis fluid through the second,
- means (b) constituting the extracorporeal circuit through which the blood passes outside the said haemodialyzer,
- means (c) for preparing the dialysis fluid,
- means (d) for circulating the dialysis fluid,
- means (f) for storing the said dialysis fluid, which may be fresh and/or used,
- members for control and monitoring (h), in particular of the said means (b) and (d),
the said means a, b, c, d and f being at least partially integrated in a common assembly (D) consisting of components produced by moulding or by injection, the said components comprising at least two flexible components (14, 15) each consisting of a thin, leakproof diaphragm which is flat in general shape and comprising rigid or semi-rigid multifunctional components (10, 11, 12, 13),
characterized in that it comprises:
- means (e) for adjusting the pressure of the dialysis fluid in the said haemodialyzer (a),
- means (g) for withdrawing and measuring quantities of fluid equal to the desired quantities of ultrafiltrate, in that the said means (e) and (g) are at least partially integrated in the said common assembly (D) and in that the said flexible components (14, 15) are equipped with apertures enabling the fluids to pass through them and are situated on both sides of at least one of the said rigid or semi-rigid multifunctional components (10, 12) and between at least another two of the said components (11, 13), the said rigid or semi-rigid components controlling, between themselves and on both sides of each of the said flexible components, the blood and dialysis fluid circuits, the said flexible components providing, in particular, by interaction with the said rigid components, functions of prevention of leakage.

2. Artificial kidney according to Claim 1, characterized in that the said haemodialyzer (a) is integrated with the said common assembly (D) which comprises two flexible components (14, 15) arranged in parallel planes, situated on both sides of the said haemodialyzer, the said rigid components (10, 11, 12, 13) constituting the components for holding in place the membranes of the said haemodialyzer (a).

3. Artificial kidney according to one of claims 1 and 2, characterized in that the said flexible components (14, 15) define the limits of longitudinal portions of the blood and dialysis fluid circuits, the dialysis fluid and the blood passing, respectively and essentially, through the one component and the other.

4. Artificial kidney according to Claim 3, characterized in that the said longitudinal portions of the said blood and dialysis fluid circuits are connected to one another via at least one transverse conduit provided inside at least one of the said rigid or semi-rigid components (10, 12).

5. Artificial kidney according to any one of the preceding claims, characterized in that the said fluid circuits, the blood passing through one of the circuits and the dialysis fluid through the other, are separated from one another by a region connected to the atmosphere (37a).

6. Artificial kidney according to any one of Claims 2 to 5, characterized in that it comprises a haemodialyzer (a) of the flat membrane type, arranged between two parallel flexible components (14, 15) clamped by two rigid components (11, 13), forming a cover, onto two other rigid components (10, 12) holding in place the stack of membranes and spacers of the said haemodialyzer, the first (10) of the said components for holding in place constituting, in particular, a reservoir for the dialysis concentrate, and the second (12) of the said components for holding in place grouping together the essential members of the said blood and dialysis fluid circuits.

7. Artificial kidney according to any one of Claims 2 to 5, characterized in that it comprises a haemodialyzer (a) of the hollow-fibre bundle type housed inside a rigid component for holding in place (12 a) whose parallel opposite faces are each connected to one of the said rigid components (11, 13) via one of the said flexible components (14, 15).

8. Artificial kidney according to any one of the preceding claims, characterized in that either of the said flexible components (14, 15) constitutes the moving and active component both of the pump body (24) and of the flap valves (24 a, 24 b) and of a diaphragm-type pump, interacting with the adjacent said rigid components (10, 11, 12, 13) and with an external motor.

9. Artificial kidney according to Claim 8, characterized in that the said flexible component (14, 15) is equipped with holes (24 a, 24 b) at the flap valves, so that the fluid displaced can pass through it.

*Fig.1.*

*Fig.2.*

*Fig.3.*

Fig.4.

Fig.5.

Fig.6.

*Fig .7.*

Fig 8.

Fig.9.

Fig. 10.

Fig. 11.

Fig.12.